# EUROPEAN PATENT SPECIFICATION

(11) **EP 1 440 143 B1**
(45) Date of publication and mention of the grant of the patent: **28.06.2006**
(21) Application number: 02770114.3
(22) Date of filing: 25.10.2002
(51) Int. Cl.: C12M 3/00, C12N 5/00, C12N 5/06, G01N 33/48, G01N 33/50

(54) **DEVICE WITH RECESSED TRACKS FOR FORMING A CELLULAR NETWORK**
VORRICHTUNG MIT VERTIEFUNGEN FÜR DIE HERSTELLUNG EINES ZELLULAREN NETZWERKES
DISPOSITIF AVEC PISTES EN RETRAIT POUR FORMATION DE RESEAU CELLULAIRE

(30) Priority: 30.10.2001 GB 0126045
(43) Date of publication of application: 28.07.2004
(73) Proprietor: Qinetiq Limited, London, SW1E 6PD (GB)
(72) Inventor: HEAL, Richard David Albert, c/o QinetiQ Winfrith, Dorchester, Dorset DT2 8XJ (GB); YEUNG, Chi-Kong, Tuen Mun, Hong Kong (CN); PARSONS, Alan Thomas, c/o QinetiQ Winfrith, Dorchester, Dorset DT2 8XJ (GB)
(74) Representative: Bowdery, Anthony Oliver
(86) International application number: PCT/GB2002/004862
(87) International publication number: WO 2003/038030

(56) References cited:
- MRKSICH MILAN ET AL: "Controlling cell attachment on contoured surfaces with self-assembled monolayers of alkanethiolates on gold." PROCEEDINGS OF THE NATIONAL ACADEMY OF SCIENCES OF THE UNITED STATES, vol. 93, no. 20, 1996, pages 10775-10778, XP002228112 1996 ISSN: 0027-8424
- GROSS G W ET AL: "ODOR, DRUG AND TOXIN ANALYSIS WITH NEURONAL NETWORKS IN VITRO: EXTRACELLULAR ARRAY RECORDING OF NETWORK RESPONSES" BIOSENSORS & BIOELECTRONICS, ELSEVIER SCIENCE PUBLISHERS, BARKING, GB, vol. 12, no. 5, 1997, pages 373-393, XP001009727 ISSN: 0956-5663
- YEUNG C K ET AL: "Modulation of the growth and guidance of rat brain stem neurons using patterned extracellular matrix proteins." NEUROSCIENCE LETTERS, vol. 301, no. 2, 30 March 2001 (2001-03-30), pages 147-150, XP002228113 ISSN: 0304-3940 cited in the application

## Description

### BACKGROUND OF THE INVENTION

The present invention relates to devices for forming cellular networks, in particular to devices for forming organised cellular networks such as organised cellular networks of neurons. The present invention also relates to methods for manufacturing and using such devices.

Structures for forming organised cellular networks or networks of cells are known. Typically, the structures have a number of pre-determined sites at which cells can form. The structures can be used to form or grow artificially assembled networks of cells disposed at the pre-determined sites.

Cellular networks which can be formed on such structures include networks of dissociated cells or networks of whole tissue cells. Example networks include networks of skin cells, networks of neurons and networks of cardiac myocytes.

Once formed, the organised cellular networks can be used in a variety of studies. These include the study of cell to cell communication, the study of compromised networks and the study of changes in the activity of cellular networks under different chemical or physiological conditions.

One particular area of interest is the formation and study of organised cellular networks of neurons or neuronal cells on defined patterns. This is referred to as neuronal cell patterning and the networks are known as "neural nets". Neural nets can be used for electrophysiological recordings, neurophysiology and the study of biochemical and molecular aspects of neuronal functions as a whole.

Yeung et al. describe the outgrowth of neurons, neurites and filopodia from brain stem slices cultured on extracellular matrix (ECM) protein structures of grid- and line-shapes. Microstamps are inked with laminin, an ECM protein, and transferred onto a substrate to form a printed pattern on the surface of the substrate. The printed pattern defines tracks for the growth of axonal connections between neurons and sites at which the neurons can become localised. The laminin acts as a chemical guidance cue for the growth and migration of the neurons. Scholl et al. describe the use of a chemical guidance cue, an adhesion peptide; in producing patterned substrates on a silicon oxide surface using micro contact printing. Tisay et al. describe the growth and guidance of neurite outgrowth using extracellular matrix constituents.

The approach of using a printed chemical guidance cue to guide formation of the cellular network suffers from several problems and disadvantages. A significant technical problem is that the cell connections tend to cross the regions of the structure that are not treated with the chemical guidance cue. It has been put forward that this may be due to slight damage or imperfections in the printed guidance cue or due to contamination of these regions with the chemical. Further, it has been observed that the cells and cell connections preferentially adhere to slightly damaged areas on the structure which may lie in the untreated regions. Therefore the formation of the cell connections along the tracks and the placement of the cells at the predetermined sites can not be relied upon. This lack of reliability means that the desired network may not be formed. Further the results are inconsistent and not reproducible. This lack of reliability, inconsistency and reproducibility represent significant disadvantages. In particular, these represent significant disadvantages in neurophysiological studies.

Neuronal organisation is generally desirable in neuronal studies. However, a problem is that perfectly patterned/organised neuronal cultures are difficult to achieve. It is also desirable to produce continuous networks of neurons. However, producing continuous networks of neurons is difficult.

Multimicroelectrode plates coated with neuronal networks for drug toxicity and screening are described by G. W. Gross et al, Biosensors and Bioelectronics, vol 12, pp373-393 (Elsevier, 1997). 64 microelectrodes with conducting patterns were photoetched onto a glass plate , insulated with polysiloxane (hydrophobic) resin and deinsulated with a laser beam. Flaming through masks allowed the generation of ahhesion patterns. Polylysine and laminin, used to enhance cell attachment and growth, attach only to flamed resin.

Embodiments of the present invention seek to mitigate problems and disadvantages associated with known approaches to forming cellular networks.

### SUMMARY OF THE INVENTION

Particular and preferred aspects of the invention are set out in the accompanying independent and dependent claims. Combinations of features from the dependent claims may be combined with features of the independent claims as appropriate and not merely as explicitly set out in the claims.

According to an aspect of the invention, there is provided a device for forming a cellular network, the device comprising:
a substrate;
a plurality of hydrophilic sites provided on the substrate for occupation by cells;
a plurality of hydrophilic tracks provided on the substrate for guiding formation of cell connections between the hydrophilic sites; and
a plurality of hydrophobic regions provided on the substrate;
wherein the hydrophilic sites and hydrophilic tracks are recessed between the hydrophobic regions.

The provision of a physical discrimination (recessing) between the tracks/sites and the regions between the tracks and sites combined with the difference in hydrophilicity represents a radical departure from known approaches to providing structures for forming cellular networks, and a device in accordance with this aspect of the invention substantially mitigates the technical problem of cell connections crossing the regions between the tracks and sites.

Advantageously, formation of cellular networks on a device in accordance with this aspect of the invention is superior to formation of networks on known structures in terms of reliability, consistency and reproducibility. Consequently, there will be an associated reduction in the time and money spent in culturing these networks.

A further advantage is that the device is versatile and can be used for the formation of many types of cellular network. The cellular network may be a network of dissociated cells or of whole tissue cells. In addition, the device provides flexibility in that any configuration (shape or pattern) or size can be achieved to suit the requirements of a range of investigations.

Further, the networks of neurons formed on the device are superior in terms of organisation and continuity. Hence, neural net production on the device can provide a versatile means for neurological investigations. One of the main areas of interest is the study of memory function and synaptic formation. Advantageously, the networks provide a structured environment for the study of how memory can be achieved, enhanced and modified. The superiority of the devices means that new experiments in this field can be devised. In addition, the superior cellular networks formed, particularly of nervous tissues, will be of use to researchers in the fields of brain function and neurological disease.

In one embodiment the hydrophilic sites and/or hydrophilic tracks are provided with a guidance cue for guiding formation of the cellular network. The guidance cue may be a physical guidance cue, achieved, for example, by placing an electric charge on the sites and/or tracks. However, preferably the guidance cue is a chemical guidance cue, such as an extracellular matrix constituent or protein. In a particular embodiment the chemical guidance cue is laminin. In other embodiments the chemical guidance cue is polylysine.

Advantageously, this chemotaxia (i.e. use of a chemical guidance cue) further enhances the reliability, consistency and reproducibility of networks formed on the device.

The hydrophobic regions may be treated with an avoidance cue to inhibit the formation of cell connections across the hydrophobic regions. Generally, the avoidance cue enhances the hydrophobicity of the hydrophobic regions. Examples include proteins such as NI-35 from myelin.

The hydrophilic sites, hydrophilic tracks and hydrophobic regions are provided on the substrate so that the hydrophilic sites and hydrophilic tracks are recessed between the hydrophobic regions. This may be achieved in any suitable way. However, preferably the substrate bears a hydrophilic layer and the plurality of hydrophobic regions are provided on the hydrophilic layer so as to define the hydrophilic sites and the hydrophilic tracks. The substrate may bear a hydrophilic layer as part of the substrate itself. In other words the substrate itself is formed from hydrophilic material. Alternatively or additionally, the hydrophilic layer may be provided as a layer of hydrophilic material provided on the substrate. An example of suitable hydrophilic material is gold. Another example is aminoalkylsilane. Suitably, the hydrophilicity of the hydrophilic material may be enhanced, for example by the chemical guidance cue.

Preferably the substrate material is transparent. Advantageously, the transparency assists in studying the networks formed on the device, for example in orienting the device with respect to analytical devices such as an electrode array. Examples of suitable substrate materials include glass, fused silica and silicon.

It will be appreciated that the layer of hydrophilic material need not be a continuous layer, so long as the layer provides the tracks and sites. For example, the layer could be discontinuous in areas that fall beneath the hydrophobic regions.

In one embodiment the hydrophobic material used for the hydrophobic regions is UV cured epoxy or photoresist. Teflon and alkylsilane are examples of other hydrophobic materials.

Suitably, one or more adhesive agents may be provided to bind the hydrophilic layer to the substrate and to bind the hydrophobic regions to the hydrophilic layer as appropriate. An example of an adhesive agent is chrome, which can be used to adhere gold to glass for example.

Typically, the hydrophilic tracks form a network of tracks connecting the hydrophilic sites. The hydrophilic tracks may extend from one side of the device to another side, although this is not necessary. In one embodiment the device comprises a sequence of parallel tracks extending in one direction and another sequence of parallel tracks extending in another direction. The hydrophilic sites may be formed at any position along the tracks, as in the line-shaped patterns described in Yeung et al., although the hydrophilic sites are preferably formed at intersections between the hydrophilic tracks. An advantage of this configuration is that the network can form or migrate in more than one direction. In a particular embodiment sequences of tracks are formed substantially perpendicular to one another. However, in other embodiments tracks may be provided in any orientation forming a network of tracks and sites as desired.

In one embodiment the hydrophilic tracks and the hydrophilic sites are recessed between the hydrophobic regions to a depth small enough for a cell to occupy a site and partially project above the surface of the hydrophobic region and deep enough for cell connections not to form across the hydrophobic regions. In other embodiments the tracks and sites are such that the cells do not partially project above the surface.

Preferably the device is configured for the formation of networks of neurons. In certain embodiments, the hydrophilic tracks and the hydrophilic sites are recessed between the hydrophobic regions to a depth in the range of 5µm to 10µm, the hydrophilic tracks have a width in the range of 6µm to 10µm, and the hydrophilic sites have a diameter in the range of 8µm to 14µm. These values have been found to be advantageous in the reliable formation of networks of neurons. A track width of 6µm has been found to be particularly preferable.

Preferably the hydrophilic sites are provided at a distance in the range of 50µm to 100µm from neighbouring hydrophilic sites. These values have been found to be particularly advantageous in the reliable formation of networks of neurons.

According to another aspect of the invention, there is provided a kit comprising a device as defined above together with and an electrode array. Suitably, the electrode array is for use in analysing the electrophysical characteristics of a cellular network formed or grown on the device. Typically, the electrode array is a complementary electrode array in that it is configured with an array of electrodes which correspond to a number of the sites provided on the device. In one embodiment the electrode array is a microelectrode array for analysing a cellular network of neurons.

According to another aspect of the invention, there is provided a method of manufacturing a device for forming a cellular network, the method comprising the steps of:
providing a plurality of hydrophilic sites on a substrate for occupation by cells;
providing a plurality of hydrophilic tracks on the substrate for guiding the formation of cell connections between the hydrophilic sites; and
recessing the hydrophilic sites and hydrophilic tracks between hydrophobic regions.

In one embodiment, the method comprises applying a guidance cue, preferably a chemical guidance cue, to the hydrophilic sites and/or the hydrophilic tracks.

In a preferred embodiment the method comprises the steps of:
(a) depositing a layer of hydrophilic material on the substrate; and
(b) depositing hydrophobic regions on the layer of hydrophilic material, so as to define the plurality of hydrophilic sites and the plurality of hydrophilic tracks on the substrate, and to recess the hydrophilic sites and hydrophilic tracks between the hydrophobic regions.

The hydrophilic material can be formed as a true layer or a discontinuous layer. An advantage of forming the hydrophilic material as a true layer is the ease of depositing a layer of material, particularly on the scale required for certain embodiments.

It will be appreciated that this is not the only way in which a device in accordance with an embodiment of the present invention may be manufactured. For example, in a modification of the preferred embodiment above, the substrate may have a hydrophilic surface forming a hydrophilic layer, and the method may comprise the step of depositing hydrophobic regions on the hydrophilic layer so as to define the plurality of hydrophilic sites and the plurality of hydrophilic tracks on the substrate, and to recess the hydrophilic sites and hydrophilic tracks between the hydrophobic regions. In this modification the hydrophilic layer takes the place of the layer of hydrophilic material.

In preferred embodiments, the deposition step (b) may comprise the steps of:
(i) depositing a layer of hydrophobic material on the layer of hydrophilic material; and
(ii) removing portions of the hydrophobic material to expose corresponding portions of the hydrophilic material, the corresponding portions of the hydrophilic material forming the hydrophilic sites and the hydrophilic tracks, and the unremoved hydrophobic material forming the hydrophobic regions.

As with the hydrophilic material, an advantage of forming the hydrophobic material as a layer is the ease of depositing a layer of material, particularly on the scale required in some embodiments.

In one embodiment, the step (ii) of removing portions of the hydrophobic material to expose corresponding portions of the hydrophilic material comprises:
applying a mask to the layer of hydrophobic material; and
subjecting the hydrophobic material to radiation through the mask to remove the portions of hydrophobic material.

Advantageously, using a mask gives particularly accurate results. The radiation is preferably electromagnetic radiation. One example uses UV radiation.

Those skilled in the art will appreciate that there are several ways of implementing a method in accordance with embodiments of the present invention. For example, instead of depositing a layer of hydrophobic material and etching it with a mask and radiation, the method may comprise:
(i) applying a mould to the layer of hydrophilic material; and
(ii) depositing hydrophobic material in the mould to form the hydrophobic regions.

Preferably, depositing hydrophilic material on the substrate to form a hydrophilic layer and/or said depositing said hydrophobic regions on the hydrophilic layer is performed by spin coating. Spin coating is particularly advantageous since it provides consistent and accurate layers of material. Optionally, the depositing may be performed by evaporation.

In some embodiments the substrate may have a hydrophobic surface and in such embodiments the method of manufacture may be modified to comprise the steps of:
recessing a plurality of sites and a plurality of tracks on the substrate, thereby providing the sites and tracks between hydrophobic regions; and
depositing hydrophilic material on the sites and tracks, thereby providing the plurality of hydrophilic sites and the plurality of hydrophilic tracks.

It will be appreciated that methods in accordance with embodiments of the present invention may be used to manufacture a device as defined above.

According to another aspect of the present invention, there is provided a method for forming a cellular network, the method comprising:
contacting a source of cells to a device as defined above; and
treating the cells to promote formation of a cellular network on the device.

In one embodiment the cells are treated with a cell feeding medium to promote formation of the cellular network. In certain embodiments the source of cells is a source of neurons.

According to another aspect of the present invention, there is provided a method for analysing a cellular network, the method comprising using an electrode array to investigate characteristics of the cells of a cellular network formed on a device as defined above. Advantageously, the superiority in terms of organisation and continuity of the cellular network means that the cells should occupy sites which correspond to the electrodes of the electrode array. Hence, analysis using an electrode array is more efficient.

The electrode array may be a microelectrode array and the cells of the cellular network may be neurons.

According to another aspect of the present invention, there is provided an assay method for identifying substances that affect the activity of a cellular network, the method comprising:
providing a cellular network with a candidate substance, the cellular network formed on a device as defined above; and
determining whether the candidate substance affects the activity of the cellular network.

Advantageously, this assay method can be used to screen hundreds of candidate substances.

The assay method may comprise the step of applying the candidate substance to the cellular network after the cellular network has been formed. Alternatively or additionally, the assay method may comprise the step of comparing the activity of the cells of the cellular network with the activity of a control cellular network to determine whether the candidate substance affects the activity of the cellular network.

Further, the assay method may comprise using an electrode array, for example a microelectrode array, to determine whether the candidate substance affects the activity of the cellular network. In one embodiment the cellular network is a network of neurons.

It will be appreciated that methods in accordance with the present invention will be of interest to Universities, the Pharmaceutical Industry, particularly in neuropharmacology and drug screening, and other research institutions.

### BRIEF DESCRIPTION OF THE DRAWINGS

Preferred embodiments of the present invention will be described hereinafter, by way of example only, with reference to the accompanying drawings in which like reference signs relate to like elements and in which:
Figures 1A to 1E schematically represent stages in a manufacture process to form a device in accordance with an embodiment of the invention and viewed from the side;
Figure 1F illustrates the device of Figure 1E in plan view;
Figures 2A to 2E schematically represent stages in a modified manufacture process to form a device in accordance with an embodiment of the invention and viewed from the side;
Figure 3 schematically illustrates a plan view of a device in accordance with an embodiment of the invention;
Figure 4A illustrates the device of Figure 3 together with a schematic representation of a source of cells on the device;
Figure 4B schematically illustrates an expanded view of a portion of the device of Figure 4A;
Figure 4C schematically illustrates an expanded view of the source of cells of Figure 4A;
Figure 5 schematically illustrates the formation of the cellular network on the device of Figures 3 and 4; and
Figure 6 schematically illustrates a device in accordance with an embodiment of the invention viewed from the side, with cells located on the device and an electrode array in contact with the cells.

### DETAILED DESCRIPTION OF PREFERRED EMBODIMENTS

Preferred embodiments of the present invention will now be described with reference to the accompanying drawings.

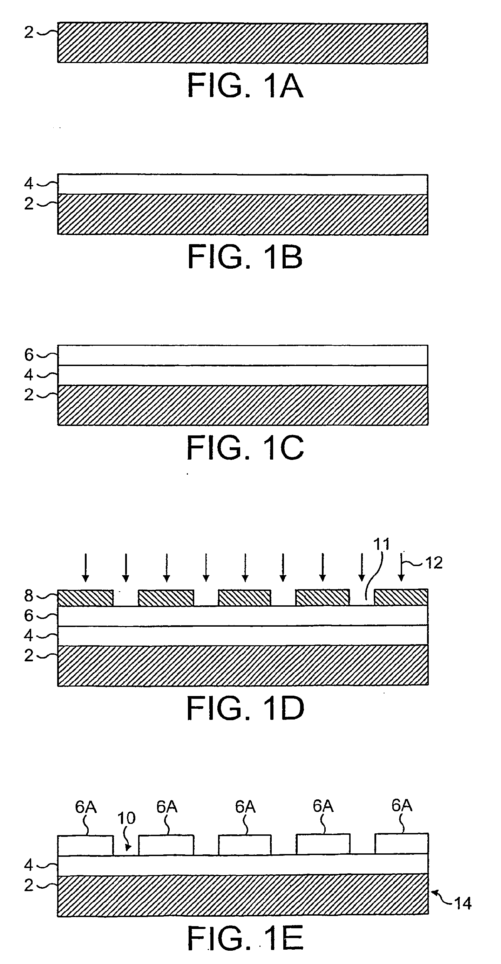

Figures 1A to 1E illustrate the stages in a process to manufacture a device for forming a cellular network. The Figures show the construction of the device in layers viewed from the side.

Referring to Figures 1A to 1E, a substrate 2 is shown in Figure 1A. A layer of hydrophilic material 4 is deposited on the substrate 2, as shown in Figure 1B. Next, a layer of hydrophobic material 6 is deposited on the layer of hydrophilic material 4 to form the intermediate structure illustrated in Figure 1C.

A mask 8 is positioned above the hydrophobic layer 6 as shown in Figure 1D. The mask 8 is preferably substantially grid-shaped in plan view (not shown) and defines a series of tracks and sites. Gaps 11 in the mask 8 defining the tracks and sites are illustrated. The mask 8 is substantially transparent to radiation in the areas defining the tracks and sites (gaps 11) but substantially opaque to radiation in the areas not defining the tracks and sites. Radiation, represented by arrows 12, is directed through the mask 8 to etch out tracks and sites in the hydrophobic layer 6 to reveal the underlying hydrophilic layer 4. The tracks and sites correspond to gaps 11. In preferred embodiments the radiation is electromagnetic radiation.

The mask is removed to leave the device 14 shown in Figure 1E. The device 14 comprises a substrate 2, a hydrophilic layer 4 and hydrophobic regions 6A between which hydrophilic tracks 10 and hydrophilic sites are recessed.

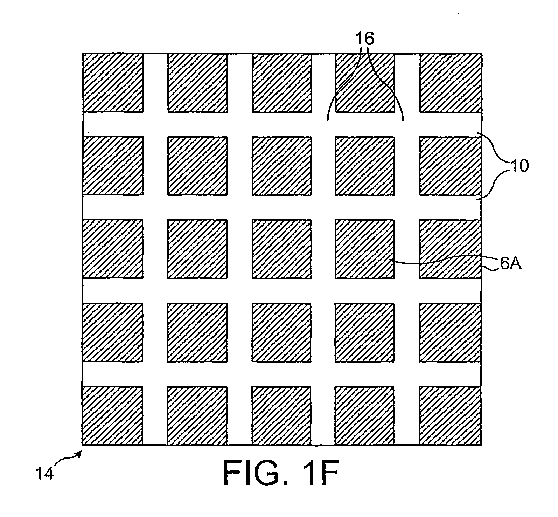

Figure 1F shows the device 14 in plan view with a series of tracks 10 (four tracks running from left to right in the Figure and four tracks running from top to bottom) and a plurality of sites 16 at the intersections between tracks 10. Hydrophobic regions 6A separating the tracks 10 and sites 16 are also shown. For clarity, only a small number of the tracks 10, sites 16 and hydrophobic regions 6A are depicted with reference signs in the Figure.

It will be appreciated that mask 8, not illustrated in plan view, has a structure which defines tracks and sites in the grid-structure illustrated in Figure 1F.

The hydrophilic material may be spin-coated or evaporated onto the substrate and the hydrophobic material may be spin-coated or evaporated onto the hydrophilic material. In particular embodiments the substrate is transparent. In a particular example the substrate is glass, the hydrophilic material is gold and the hydrophobic material is UV cured epoxy or photoresist. The gold is deposited by evaporation. The mask may be made from a UV-transparent material bearing a metal pattern and the radiation used may be UV light. Chrome may be used as an adhesive agent to adhere the gold to the glass and the UV cured epoxy or photoresist can be post-treated to increase the hydrophobicity if required.

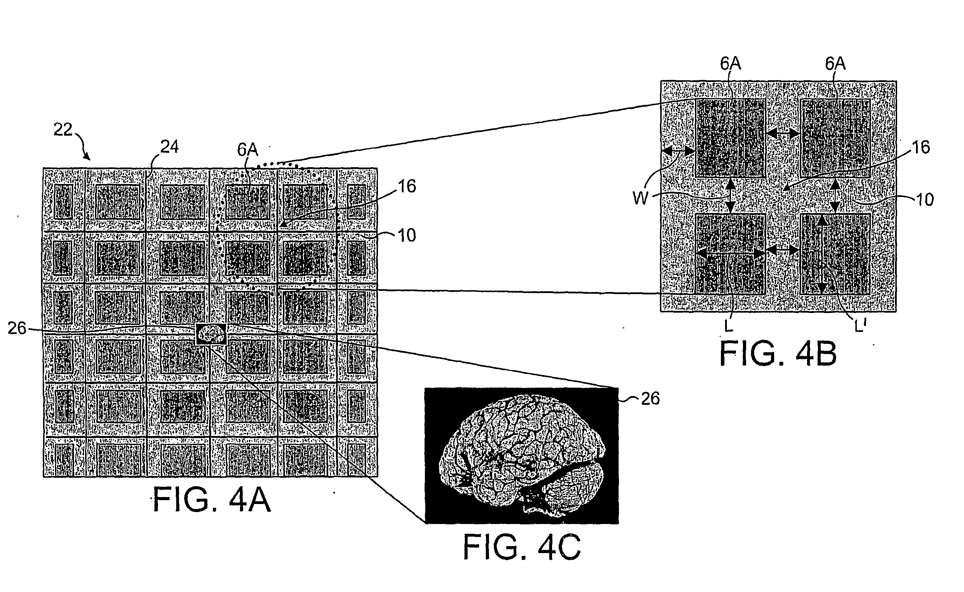

Figures 1A to 1E are schematic illustrations and are not to scale. In examples the hydrophobic layer is from 5µm to 10µm thick and the hydrophilic layer is from 1µm to 2µm thick, although the thickness of the hydrophilic layer is not an important factor and any suitable thickness can be used. Preferably, the hydrophilic layer is not so thick that it has poor optical properties and not so thin that the layer breaks up upon contact with the cells. The tracks may preferably be from 6µm to 10µm wide (see width w, Figure 4B) and the width (l, Figure 4B) and length (l', Figure 4B) of the hydrophobic regions may preferably be from 50µm to 100µm.

The devices in these examples are typically of the order of 1cm long and 1cm wide with 75 to around 150 tracks running from one side of the device to the other. Other examples are longer and wider with larger numbers of tracks. Other examples are smaller with fewer tracks. Other examples have different shapes and patterns.

Figure 1F illustrates the sites 16 as being defined by square hydrophobic regions with pointed corners. However, this is a schematic representation and the hydrophobic regions preferably have rounded or blunted corners to provide a site for occupation by the cells of the cellular network. In particular examples the diameter of the sites, i.e. the measurement across a site between diagonally opposed hydrophobic regions, is between 8µm and 14µm.

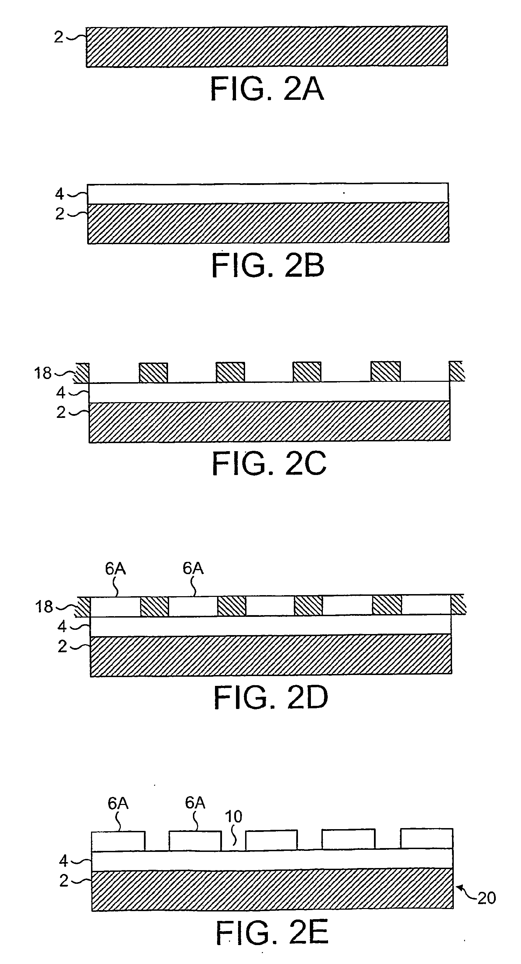

Figures 2A to 2E illustrate the stages in a modified process to manufacture a device for forming a cellular network. Like Figures 1A to 1E, these Figures show the construction of the device in layers viewed from the side.

Referring to Figures 2A to 2E, Figures 2A and 2B correspond to Figures 1A and 1B and a layer of hydrophilic material 4 is deposited on the substrate 2. Next, a mould 18 is positioned on the hydrophilic layer 4 as shown in Figure 2C.

The mould 18 is substantially grid-shaped in plan view (not shown) and defines regions between a series of tracks and sites. The ends of the mould 18 are illustrated. Hydrophobic material is deposited into the regions to form hydrophobic regions 6A as illustrated in Figure 2D. The hydrophobic material is only deposited in the regions between the tracks and sites and not in the tracks and sites themselves. The mould 18 is removed to leave the device 20 shown in Figure 2E.

As can be seen, the structure of device 20 is identical to that of device 14 shown in Figure 1E. Device 20 comprises a substrate 2, a hydrophilic layer 4 and hydrophobic regions 6A between which hydrophilic tracks 10 and hydrophilic sites are recessed. The structure of device 20 in plan view is the same as that of device 14 shown in Figure 1E.

It will be appreciated that mould 18, not illustrated in plan view, has a structure which defines regions between tracks and sites in the grid-structure illustrated in Figure 1F. In a preferred embodiment the hydrophobic material is poured into mould 18 and allowed to set. Examples of devices manufactured using the modified process of Figures 2A to 2E have the same dimensions as those given above for devices formed using the process of Figures 1A to 1E.

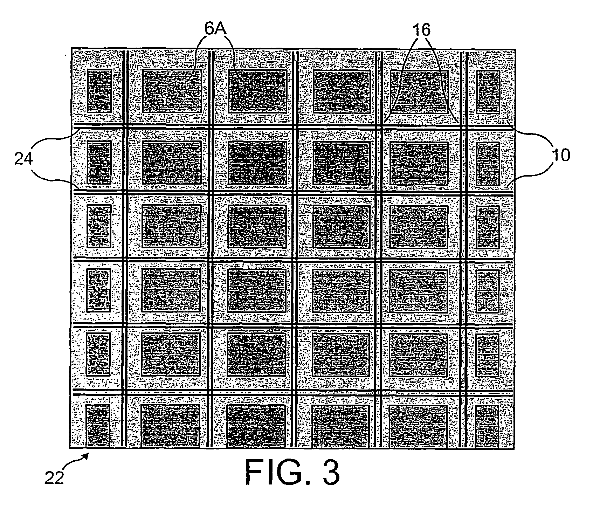

Figure 3 is a schematic plan view of a device 22 for forming a cellular network. The device 22 is similar in plan view to devices 14 and 20 described above, although device 22 is illustrated with a different number of tracks and sites. Device 22 comprises a plurality of hydrophilic tracks 10 and a plurality of hydrophilic sites 16. The tracks 10 and sites 16 are recessed between a plurality of hydrophobic regions 6A. The dimensions and materials described above in relation to devices 14 and 20 similarly apply to device 22. However, the hydrophilic tracks 10 and sites 16 of device 22 are coated with a chemical guidance cue 24.

In particular examples the chemical guidance cue is an extracellular matrix (ECM) protein such as laminin. In other examples the chemical guidance cue is polylysine. The chemical guidance cue is applied to the tracks 10 and sites 16 by pouring the chemical onto the device and allowing the chemical to flow from the hydrophobic regions into the hydrophilic areas. The chemical is then preferably blow dried.

The tracks and sites of devices 14 and 20 can be similarly coated with a chemical guidance cue.

Figures 4A to 4C, 5 and 6 relate to the use of devices in accordance with embodiments of the present invention. The devices can be used to grow networks of cells with the cells disposed at the sites on the device and with the cell connections between the cells lying along the tracks. A particular example of a network which can be grown on these devices is a network of neurons.

Referring now to Figure 4A, device 22 is shown with a source of cells 26 provided on a cell seeding area on the device. In the illustrated embodiment the cell seeding area is simply a nominated area in the centre of the device and the source of cells is positioned on the tracks and sites in this area. In other embodiments the cell seeding area is a designated area which is not provided with hydrophilic tracks and hydrophilic sites. In such embodiments the areas adjacent the designated area are provided with hydrophilic tracks and hydrophilic sites for the cellular network to form on. Preferably, the designated area is hydrophilic. The designated area may be recessed to the same depth as the hydrophilic tracks and hydrophilic sites. Optionally, the designated area may be disposed at the same level as the hydrophobic regions.

Figure 4B shows an expanded portion of the device 22 and Figure 4C shows an expanded illustration of the source of cells 26.

The source of cells is treated to promote formation of a cellular network on the device and this typically comprises treating with a cell feeding medium. In preferred embodiments, the cell seeding area 26 (not shown to scale in the Figures) is approximately 3mm wide. Typically, the device has between about 28 and about 53 tracks between the cell seeding area 26 and the sides of the device.

In a preferred embodiment the source of cells 26 is a source of neurons. In a particular example ultra-thin brain slices are used as the source of cells. As described by Yeung et al. in connection with the growth of cells on a typical prior art structure, coronal sections of 250µm thick rat brain slices are harvested in sterile conditions in chilled brain slice culture medium (pH 7.4) made from HAMS F10 supplemented with 20-25% foetal bovine serum and 4mM glutamine, all from SIGMA. The prepared slices are placed into an incubator at 37°C and at a 5% CO₂ enriched atmosphere for 4-5 hours minimum. After the incubation period, the brain slices are positioned onto sterilised devices and treated with a critical amount of brain slice culture medium. After 2-3 days in culture, the slices undergo significant migration and more medium is added. After 2 weeks the axons reach maturity and after 3 weeks the neurons and their processes migrate over 1000-2000µm of uninterrupted patterns.

It will be appreciated that for this kind of use, the device should be able to tolerate sterilisation which may involve submerging the device in 70% alcohol or heating the device to 120°C for a minimum of 20 minutes. Further, the device should be able to withstand exposure to a temperature range of between 4°C to 40°C and a pH range of between 6.5 - 7.9.

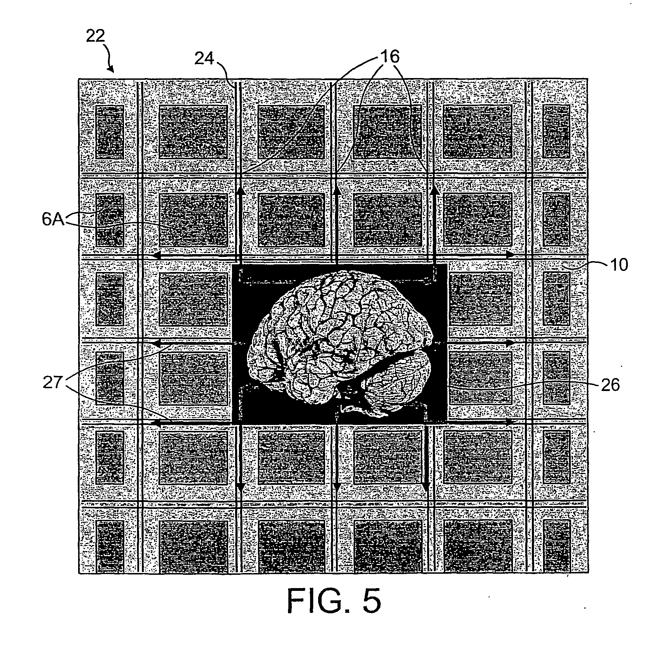

The migration/formation of the cellular network of cells in the sites 16 is schematically depicted in Figure 5. The arrows 27 represent the migration of cells to the sites 16. The tracks 10 guide formation of cell connections between the sites 16 and the cells occupy the sites 16. Cell migration from one site 16 to another site 16 also occurs, as does the formation of cell connections between sites occupied by cells, although these processes are not illustrated in the schematic depiction of Figure 5.

Once the cellular network has been formed on the device, the cellular network can be used in a variety of studies. These include the study of the cellular network using electrode arrays comprising electrodes which are spaced to correspond to the spacing between the sites of the device and hence to the spacing between the cells of the cellular network. The electrode array can be put in contact with a number of the cells of the network and used to stimulate one or more cells and to detect responses at one or more of the other cells.

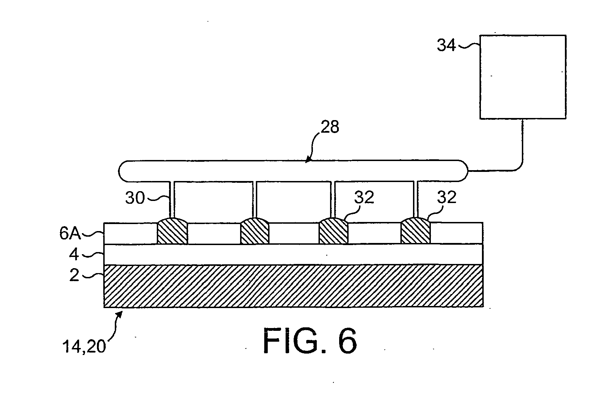

Figure 6 schematically illustrates an electrode array 28 comprising a plurality of electrodes 30. The electrodes 30 are brought into contact with the cells 32 formed on the device 14, 20. The electrode array 28 is connected to controller 34 which is used to stimulate and detect activity in the cells. The electrical activity of the cellular network is thus measured.

As can be seen from Figure 6, the cells 32 preferably sit proud of the surface of the device 14, 20 so that the electrode array 28 can be brought into contact with them, although this is not necessary as electrode arrays which can contact cells which do not sit proud of the surface can be used. In embodiments with a transparent substrate, the transparency assists in orienting the electrode array in relation to the cellular network. In certain circumstances the cellular network can be removed from the device before analysis of the network commences.

Any cellular networks that have a fluctuating electrical gradient across the cell membrane can be studied using electrode arrays, for example networks of heart cells or neurons. An example of an electrode array is a microelectrode array. Microelectrode arrays can be used to study the properties of networks of neurons. Gross et al. describe odor, drug and toxin analysis with neuronal network in vitro and extracellular array recording of network responses.

Devices in accordance with embodiments of the present invention may be formed with the sites and tracks in any desired configuration. Electrode arrays which have electrodes which are spaced to correspond to the spacing between the sites of the device may then be provided. One or more of the devices and one or more complementary electrode arrays may be packaged together as a kit.

One particularly useful study in which the devices can be used is in an assay for identifying substances that affect the activity of a cellular network. A cellular network can be provided with a candidate substance by for example applying the substance to the formed cellular network. The activity of the cellular network can be analysed before and after application of the candidate substance to determine whether there is an associated change in the activity of the network. The electrical activity of the cellular network is measured and any temporal changes in spike/burst rates and changes in the signal shapes are analysed.

Rather than applying the candidate substance to the formed network, the substance may be applied by pre-treating the source of cells with the substance.

In certain embodiments an electrode array is used to determine whether the activity of the cellular network is affected by the candidate substance. In one embodiment the activity of the cells of the cellular network to which the candidate substance has been applied is compared with the activity of a control cellular network to determine whether the candidate substance affects the activity of the cellular network.

In a particular example the assay is used to analyse a cellular network of neurons. Thus candidate substances to treat neuronal disorders and conditions can be identified. Such disorders and conditions include memory loss (such as that which occurs in Parkinsonism and Depression), Cerebral ischaemia, and toxin-induced axonal/neuronal damage (for example from toxins in air or water).

It will be appreciated that cellular networks formed on devices in accordance with embodiments of the present invention may be used as a primary screen for drug screening. An array of networks may be probed with several hundred candidate substances to see whether there is an associated change in the activity of the network and whether the change is desirable.

Although the invention has been described in relation to the preceding example embodiments, it will be appreciated that the invention is not limited thereto, and that many variations are possible falling within the scope of the invention as defined in the appended claims.

Example variations within the scope of the present invention include using a physical guidance cue instead or as well as a chemical guidance cue. An example of a physical guidance cue is a charge provided on the tracks and sites. Chemical guidance cues other than ECM proteins may also be used, for example collagen, polyethyleneimine, poly-L-lysine and poly-D-lysine. Further, the substrate itself may have a hydrophilic surface which can perform the same function as the layer of hydrophilic material. The layer of hydrophilic material may be discontinuous in some areas, in particular areas which are disposed beneath the hydrophobic regions.

Further variations include forming the hydrophilic sites at positions on the tracks which are not at intersections between tracks. An example of such a configuration is described in Yeung et al. where line-shaped tracks are used with sites disposed along the tracks. Devices having any desired configuration of tracks and sites can be formed.

The substrate material, hydrophilic material and hydrophobic material may be any suitable materials. Examples of suitable substrate material include plastic and silicon. Optionally, the materials may be bound by any suitable adhesive agent.

The hydrophobic regions may be provided with an avoidance cue, for example a protein such as NI-35 from myelin, to inhibit formation of cell connections across these regions.

Methods of manufacture within the scope of the present invention include, where the substrate has a hydrophilic layer, depositing hydrophobic regions on the hydrophilic layer. Where the substrate has a hydrophobic surface, the method includes recessing the sites and tracks on the substrate and depositing hydrophilic material on the sites and tracks.

Variations in the steps of methods of manufacture also include using other processes to form the layers and recesses on the device. These may include epitaxy, photolithography, laser etching and electron beam etching.

Variations within the scope of the assay method include using other techniques to study the activity of the cellular network. For example, patch clamp (intracellular) recording may be used. Multiple-patch clamp recording may also be used, in particular to study neuronal functions. Another suitable technique is calcium imaging such as Furo-3.

For the avoidance of doubt the term "comprising", as used throughout the description and claims is not to be construed solely as meaning "consisting only of".

### REFERENCES

Yeung et al., Modulation of the growth and guidance of rat brain stem neurons using patterned extracellular matrix proteins, Neuroscience Letters Vol. 301 (2001), pages 147-150.

Scholl et al., Ordered networks of rat hippocampal neurons attached to silicon oxide surfaces, J. Neuroscience Methods, 104(1) (2001), pages 65-75.

Tisay et al., The extracellular matrix modulates olfactory neurite outgrowth on ensheathing cells, J. Neuroscience, 19 (22) (1999), pages 9890-9899.

Gross et al., Odor, drug and toxin analysis with neuronal networks in vitro: extracellular array recording of network responses, Biosensors & Bioelectronics, 12(5) (1997), pages 373-393.

## Claims

1. A device for forming a cellular network, the device comprising:
a substrate;
a plurality of hydrophilic sites provided on the substrate for occupation by cells;
a plurality of hydrophilic tracks provided on the substrate for guiding formation of cell connections between the hydrophilic sites; and
a plurality of hydrophobic regions provided on the substrate;
wherein the hydrophilic sites and hydrophilic tracks are recessed between the hydrophobic regions.

2. A device as claimed in claim 1, wherein the hydrophilic sites and/or hydrophilic tracks are provided with a guidance cue for guiding formation of the cellular network.

3. A device as claimed in claim 2, wherein the guidance cue is a chemical guidance cue.

4. A device as claimed in claim 3, wherein the chemical guidance cue is an extracellular matrix protein.

5. A device as claimed in any one of claims 1 to 4, wherein the substrate bears a hydrophilic layer and the plurality of hydrophobic regions are provided on the hydrophilic layer so as to define the hydrophilic sites and the hydrophilic tracks.

6. A device as claimed in claim 5, wherein the hydrophilic layer is a layer of hydrophilic material provided on the substrate.

7. A device as claimed in any one of claims 1 to 6, wherein the substrate is substantially transparent.

8. A device as claimed in any one of claims 1 to 7, wherein the hydrophilic sites are formed at intersections between the hydrophilic tracks.

9. A device as claimed in any one of claims 1 to 8, wherein the hydrophilic tracks and the hydrophilic sites are recessed between the hydrophobic regions to a depth small enough for a cell to occupy a site and partially project above the surface of the hydrophobic region and deep enough for cell connections not to form across the hydrophobic regions.

10. A device as claimed in any one of claims 1 to 9, wherein the hydrophilic tracks and the hydrophilic sites are recessed between the hydrophobic regions to a depth in the range of 5µm to 10µm.

11. A device as claimed in any one of claims 1 to 10, wherein the hydrophilic tracks have a width in the range of 6µm to 10µm.

12. A device as claimed in any one of claims 1 to 11, wherein the hydrophilic sites have a diameter in the range of 8µm to 14µm.

13. A device as claimed in any one of claims 1 to 12, wherein the hydrophilic sites are provided at a distance in the range of 50µm to 100µm from neighbouring hydrophilic sites.

14. A kit comprising a device according to any one of claims 1 to 13 and an electrode array.

15. A method of manufacturing a device for forming a cellular network, the method comprising the steps of:
providing a plurality of hydrophilic sites on a substrate for occupation by cells;
providing a plurality of hydrophilic tracks on the substrate for guiding the formation of cell connections between the hydrophilic sites; and
recessing the hydrophilic sites and hydrophilic tracks between hydrophobic regions.

16. A method as claimed in claim 15, further comprising:
applying a guidance cue to the hydrophilic sites and/or the hydrophilic tracks.

17. A method as claimed in claim 16, wherein the guidance cue is a chemical guidance cue.

18. A method as claimed in any one of claims 15 to 17, comprising the steps of:
(a) depositing a layer of hydrophilic material on the substrate; and
(b) depositing hydrophobic regions on the layer of hydrophilic material, so as to define the plurality of hydrophilic sites and the plurality of hydrophilic tracks on the substrate, and to recess the hydrophilic sites and hydrophilic tracks between the hydrophobic regions.

19. A method as claimed in claim 18, wherein the step (b) comprises the steps of:
(i) depositing a layer of hydrophobic material on the layer of hydrophilic material; and
(ii) removing portions of the hydrophobic material to expose corresponding portions of the hydrophilic material, the corresponding portions of the hydrophilic material forming the hydrophilic sites and the hydrophilic tracks, and the unremoved hydrophobic material forming the hydrophobic regions.

20. A method as claimed in claim 19, wherein the step (ii) comprises the steps of:
applying a mask to the layer of hydrophobic material; and
subjecting the hydrophobic material to radiation through the mask to remove the portions of hydrophobic material.

21. A method as claimed in claim 18, wherein the step (b) comprises the steps of:
(i) applying a mould to the layer of hydrophilic material; and
(ii) depositing hydrophobic material in the mould to form the hydrophobic regions.

22. A method as claimed in any one of claims 18 to 21, wherein said depositing step (a) and/or said depositing step (b) is performed by spin coating.

23. A method as claimed in any one of claims 15 to 17, wherein the substrate has a hydrophilic surface forming a hydrophilic layer, the method comprising the step of:
depositing hydrophobic regions on the hydrophilic layer so as to define the plurality of hydrophilic sites and the plurality of hydrophilic tracks on the substrate, and to recess the hydrophilic sites and hydrophilic tracks between the hydrophobic regions.

24. A method as claimed in any one of claims 15 to 17, wherein the substrate has a hydrophobic surface, the method comprising:
recessing a plurality of sites and a plurality of tracks in the substrate, thereby providing the sites and tracks between hydrophobic regions; and
depositing hydrophilic material on the sites and tracks, thereby providing the plurality of hydrophilic sites and the plurality of hydrophilic tracks.

25. A method for forming a cellular network, the method comprising:
contacting a source of cells to a device according to any one of claims 1 to 13; and
treating the cells to promote formation of a cellular network on the device.

26. A method as claimed in claim 25, comprising the step of treating the cells with a cell feeding medium to promote formation of the cellular network.

27. A method as claimed in claim 25 or 26, wherein the source of cells is a source of neurons.

28. A method for analysing a cellular network, the method comprising using an electrode array to investigate characteristics of the cells of a cellular network formed on a device according to any one of claims 1 to 13.

29. A method as claimed in claim 28, wherein the cells of the cellular network are neurons.

30. An assay method for identifying substances that affect the activity of a cellular network, the method comprising:
providing a cellular network with a candidate substance, the cellular network formed on a device according to any one of claims 1 to 13; and
determining whether the candidate substance affects the activity of the cellular network.

31. An assay method as claimed in claim 30, the method comprising the step of applying the candidate substance to the cellular network after the cellular network has been formed.

32. An assay method as claimed in claim 30 or 31, comprising the step of comparing the activity of the cells of the cellular network with the activity of a control cellular network to determine whether the candidate substance affects the activity of the cellular network.

33. An assay method as claimed in any one of claims 30 to 32, the method comprising the step of using an electrode array to determine whether the candidate substance affects the activity of the cellular network.

34. An assay method as claimed in any one of claims 30 to 33, wherein the cellular network is a network of neurons.

## Patentansprüche

1. Vorrichtung zur Ausbildung eines zellularen Netzwerks, die umfaßt:
- ein Substrat,
- mehrere hydrophile, auf dem Substrat vorgesehene Stellen zur Belegung durch Zellen,
- mehrere hydrophile, auf dem Substrat vorgesehene Spuren zum Lenken der Ausbildung von Zellverbindungen zwischen den hydrophilen Stellen,
- mehrere hydrophobe, auf dem Substrat vorgesehene Bereiche, wobei die hydrophilen Stellen und die hydrophilen Spuren zwischen den hydrophoben Bereichen vertieft sind.

2. Vorrichtung nach Anspruch 1, bei der die hydrophilen Stellen und/oder die hydrophilen Spuren mit einer Signalstoff zum Lenken der Ausbildung des zellularen Netzwerks versehen sind.

3. Vorrichtung nach Anspruch 2, bei der der Signalstoff ein chemischer Signalstoff ist.

4. Vorrichtung nach Anspruch 3, bei der der chemische Signalstoff ein extrazellulares Matrixprotein ist.

5. Vorrichtung nach einem der Ansprüche 1 bis 4, bei der das Substrat eine hydrophile Schicht trägt und die mehreren hydrophoben Bereiche auf der hydrophilen Schicht so vorgesehen sind, dass sie die hydrophilen Stellen und die hydrophilen Spuren vorgeben.

6. Vorrichtung nach Anspruch 5, bei der die hydrophile Schicht eine auf dem Substrat vorgesehene Schicht aus hydrophilem Material ist.

7. Vorrichtung nach einem der Ansprüche 1 bis 6, bei der das Substrat im wesentlichen transparent ist.

8. Vorrichtung nach einem der Ansprüche 1 bis 7, bei der die hydrophilen Stellen an den Schnittpunkten zwischen den hydrophilen Spuren ausgebildet sind.

9. Vorrichtung nach einem der Ansprüche 1 bis 8, bei der die hydrophilen Spuren und die hydrophilen Stellen zwischen den hydrophoben Bereichen auf eine Tiefe vertieft sind, welche klein genug ist, damit eine Zelle eine Stelle besetzen kann und zum Teil über die Oberfläche des hydrophoben Bereichs hinausragt, und tief genug ist, damit sich keine Zellverbindungen quer durch die hydrophoben Bereiche bilden.

10. Vorrichtung nach einem der Ansprüche 1 bis 9, bei der die hydrophilen Spuren und die hydrophilen Stellen zwischen den hydrophoben Bereichen auf eine Tiefe im Bereich von 5 bis 10 µm vertieft sind.

11. Vorrichtung nach einem der Ansprüche 1 bis 10, bei der die hydrophilen Spuren eine Breite im Bereich von 6 bis 10 µm haben.

12. Vorrichtung nach einem der Ansprüche 1 bis 11, bei der die hydrophilen Stellen einen Durchmesser im Bereich von 8 bis 14 µm haben.

13. Vorrichtung nach einem der Ansprüche 1 bis 12, bei der die hydrophilen Stellen in einem Abstand im Bereich von 50 bis 100 µm von den benachbarten hydrophilen Stellen vorgesehen sind.

14. Kit, welches eine Vorrichtung nach einem der Ansprüche 1 bis 13 und ein Elektrodenfeld umfaßt.

15. Verfahren zur Herstellung einer Vorrichtung zur Ausbildung eines zellularen Netzwerks, welches die folgenden Schritte umfaßt:
- Vorsehen mehrerer hydrophiler Stellen auf einem Substrat zur Besetzung durch Zellen,
- Vorsehen mehrerer hydrophiler Spuren auf dem Substrat zum Lenken der Ausbildung von Zellverbindungen zwischen den hydrophilen Stellen und
- Vertiefen der hydrophilen Stellen und der hydrophilen Spuren zwischen hydrophoben Bereichen.

16. Verfahren nach Anspruch 15, welches ferner den Schritt des Aufbringens eines Signalstoffes auf die hydrophilen Stellen und/oder hydrophilen Spuren umfaßt.

17. Verfahren nach Anspruch 16, bei dem der Signalstoff ein chemischer Signalstoff ist.

18. Verfahren nach einem der Ansprüche 15 bis 17, welches die folgenden Schritte umfaßt:
(a) Auftragen einer Schicht eines hydrophilen Materials auf das Substrat und
(b) Auftragen hydrophober Bereiche auf die Schicht aus hydrophilem Material derart, dass die mehreren hydrophilen Stellen und die mehreren hydrophilen Spuren auf dem Substrat vorgegeben werden und die hydrophilen Stellen und die hydrophilen Spuren zwischen den hydrophoben Bereichen vertieft sind.

19. Verfahren nach Anspruch 18, bei dem der Schritt (b) die folgenden Schritte umfaßt:
(i) Auftragen einer Schicht eines hydrophoben Materials auf die Schicht des hydrophilen Materials und
(ii) Entfernen von Teilen des hydrophoben Materials zur Freilegung entsprechender Abschnitte des hydrophilen Materials, wobei die entsprechenden Abschnitte des hydrophilen Materials die hydrophilen Stellen und die hydrophilen Spuren ausbilden und das nicht entfernte hydrophobe Material die hydrophoben Bereiche bildet.

20. Verfahren nach Anspruch 19, bei dem der Schritt (ii) die folgenden Schritte umfaßt:
- Aufbringen einer Maske auf die Schicht aus hydrophobem Material und
- Aussetzen des hydrophoben Materials einer Bestrahlung durch die Maske zur Entfernung von Abschnitten des hydrophoben Materials.

21. Verfahren nach Anspruch 18, bei dem der Schritt (b) die folgenden Schritte umfaßt:
(i) Aufbringen einer Form auf die Schicht des hydrophilen Materials und
(ii) Einbringen eines hydrophoben Materials in die Form zur Ausbildung der hydrophoben Bereiche.

22. Verfahren nach einem der Ansprüche 18 bis 21, bei dem der Schritt (a) und/oder (b) des Auftrags durch Rotationsbeschichtung erfolgt.

23. Verfahren nach einem der Ansprüche 15 bis 17, bei dem das Substrat eine hydrophile Oberfläche aufweist, die eine hydrophile Schicht bildet, und welches den Schritt des
- Auftragens von hydrophoben Bereichen auf der hydrophilen Schicht derart umfaßt, dass die mehreren hydrophilen Schichten und die mehreren hydrophilen Spuren auf dem Substrat vorgegeben werden und die hydrophilen Stellen und die hydrophilen Spuren zwischen den hydrophoben Bereichen vertieft sind.

24. Verfahren nach einem der Ansprüche 15 bis 17, bei dem das Substrat eine hydrophobe Oberfläche aufweist und welches den Schritt der
- Vertiefung mehrer Stellen und mehrerer Spuren im Substrat umfaßt, wodurch die Stellen und die Spuren zwischen den hydrophoben Bereichen vorgegeben werden, und
- Auftrag von hydrophilem Material auf die Stellen und die Spuren, wodurch die mehreren hydrophilen Schichten und die mehreren hydrophilen Spuren erzeugt werden.

25. Verfahren zur Ausbildung eines zellularen Netzwerks, welches die Schritte umfaßt:
- Inkontaktbringen einer Quelle von Zellen mit einer Vorrichtung nach einem der Ansprüche 1 bis 13 und
- Behandlung der Zellen zur Förderung der Ausbildung eines zellularen Netzwerks auf der Vorrichtung.

26. Verfahren nach der Anspruch 25, welches den Schritt der Behandlung der Zellen mit einem Zellenfütterungsmedium zur Förderung der Ausbildung des zellularen Netzwerks umfaßt.

27. Verfahren nach Anspruch 25 oder 26, bei dem die Quelle der Zellen eine Neuronenquelle ist.

28. Verfahren zur Analyse eines zellularen Netzwerks, welches den Schritt der Verwendung eines Elektrodenfeldes zur Untersuchung der Kennwerte der Zellen eines zellularen Netzwerks umfaßt, das auf einer Vorrichtung nach einem der Ansprüche 1 bis 13 ausgebildet ist.

29. Verfahren nach der Anspruch 28, bei dem die Zellen des zellularen Netzwerks Neuronen sind.

30. Testverfahren zur Erkennung von Substanzen, welche die Aktivität eines zellularen Netzwerks beeinträchtigen, welches die folgenden Schritte umfaßt:
- Versehen eines zellularen Netzwerks mit einer zu prüfenden Substanz, wobei das zellulare Netzwerk auf einer Vorrichtung nach einem der Ansprüche 1 bis 13 ausgebildet ist, und
- Bestimmung, ob die zu prüfende Substanz die Aktivität des zellularen Netzwerks beeinträchtigt.

31. Testverfahren nach Anspruch 30, welches den Schritt der Einbringung der zu prüfenden Substanz in das zellulare Netzwerk umfaßt, nachdem dieses ausgebildet ist.

32. Testverfahren nach Anspruch 30 oder 31, welches den Schritt eines Vergleichs der Aktivität der Zellen des zellularen Netzwerks mit der Aktivität der Zellen eines zellularen Kontrollnetzwerks umfaßt, um festzustellen, ob die zu prüfende Substanz die Aktivität des zellularen Netzwerks beeinträchtigt.

33. Testverfahren nach einem der Ansprüche 30 bis 32, welches den Schritt der Verwendung eines Elektrodenfeldes zur Feststellung umfaßt, ob die zu prüfende Substanz die Aktivität des zellularen Netzwerks beeinträchtigt.

34. Testverfahren nach einem der Ansprüche 30 bis 33, bei dem das zellulare Netzwerk ein Neuronennetzwerk ist.

## Revendications

1. Dispositif destiné à la formation d'un réseau cellulaire, le dispositif comprenant :
un substrat ;
une pluralité de sites hydrophiles fournis sur le substrat pour une occupation par des cellules ;
une pluralité de pistes hydrophiles fournies sur le substrat afin de guider la formation de connexions cellulaires entre les sites hydrophiles ; et
une pluralité de régions hydrophobes fournies sur le substrat ;
dans lequel les sites hydrophiles et les pistes hydrophiles sont en retrait entre les régions hydrophobes.

2. Dispositif selon la revendication 1, dans lequel les sites hydrophiles et/ou les pistes hydrophiles sont pourvus d'un repère de guidage destiné à guider la formation du réseau cellulaire.

3. Dispositif selon la revendication 2, dans lequel le repère de guidage est un repère de guidage chimique.

4. Dispositif selon la revendication 3, dans lequel le repère de guidage chimique est une protéine de matrice extracellulaire.

5. Dispositif selon l'une quelconque des revendications 1 à 4, dans lequel le substrat porte une couche hydrophile et la pluralité de régions hydrophobes sont fournies sur la couche hydrophile de manière à définir les sites hydrophiles et les pistes hydrophiles.

6. Dispositif selon la revendication 5, dans lequel la couche hydrophile est une couche de matière hydrophile fournie sur le substrat.

7. Dispositif selon l'une quelconque des revendications 1 à 6, dans lequel le substrat est substantiellement transparent.

8. Dispositif selon l'une quelconque des revendications 1 à 7, dans lequel les sites hydrophiles sont formés aux intersections entre les pistes hydrophiles.

9. Dispositif selon l'une quelconque des revendications 1 à 8, dans lequel les pistes hydrophiles et les sites hydrophiles sont en retrait entre les régions hydrophobes à une profondeur suffisamment petite pour qu'une cellule occupe un site et fasse saillie partiellement au-dessus de la surface de la région hydrophobe et suffisamment profonde pour que des connexions cellulaires ne se forment pas à travers les régions hydrophobes.

10. Dispositif selon l'une quelconque des revendications 1 à 9, dans lequel les pistes hydrophiles et les sites hydrophiles sont en retrait entre les régions hydrophobes à une profondeur dans la plage de 5 µm à 10 µm.

11. Dispositif selon l'une quelconque des revendications 1 à 10, dans lequel les pistes hydrophiles ont une largeur dans la plage de 6 µm à 10 µm.

12. Dispositif selon l'une quelconque des revendications 1 à 11, dans lequel les sites hydrophiles ont un diamètre dans la plage de 8 µm à 14 µm.

13. Dispositif selon l'une quelconque des revendications 1 à 12, dans lequel les sites hydrophiles sont fournis à une distance dans la plage de 50 µm à 100 µm des sites hydrophiles voisins.

14. Kit comprenant un dispositif selon l'une quelconque des revendications 1 à 13 et une matrice d'électrodes.

15. Procédé de fabrication d'un dispositif destiné à la formation d'un réseau cellulaire, le procédé comprenant les étapes suivantes :
fourniture d'une pluralité de sites hydrophiles sur un substrat pour une occupation par des cellules ;
fourniture d'une pluralité de pistes hydrophiles sur le substrat afin de guider la formation de connexions cellulaires entre les sites hydrophiles ; et
mise en retrait des sites hydrophiles et des pistes hydrophiles entre les régions hydrophobes.

16. Procédé selon la revendication 15, comprenant en outre :
l'application d'un repère de guidage aux sites hydrophiles et/ou aux pistes hydrophiles.

17. Procédé selon la revendication 16, dans lequel le repère de guidage est un repère de guidage chimique.

18. Procédé selon l'une quelconque des revendications 15 à 17, comprenant les étapes suivantes :
(a) dépôt d'une couche de matière hydrophile sur le substrat ; et
(b) dépôt de régions hydrophobes sur la couche de matière hydrophile, de manière à définir la pluralité de sites hydrophiles et la pluralité de pistes hydrophiles sur le substrat, et à mettre en retrait les sites hydrophiles et les pistes hydrophiles entre les régions hydrophobes.

19. Procédé selon la revendication 18, dans lequel l'étape (b) comprend les étapes suivantes :
(i) dépôt d'une couche de matière hydrophobe sur la couche de matière hydrophile ; et
(ii) enlèvement de parties de la matière hydrophobe afin d'exposer des parties correspondantes de la matière hydrophile, les parties correspondantes de la matière hydrophile formant les sites hydrophiles et les pistes hydrophiles, et la matière hydrophobe non enlevée formant les régions hydrophobes.

20. Procédé selon la revendication 19, dans lequel l'étape (ii) comprend les étapes suivantes :
application d'un masque à la couche de matière hydrophobe ; et
exposition de la matière hydrophobe à un rayonnement à travers le masque afin d'enlever les parties de matière hydrophobe.

21. Procédé selon la revendication 18, dans lequel l'étape (b) comprend les étapes suivantes :
(i) application d'un moule à la couche de matière hydrophile ; et
(ii) dépôt de matière hydrophobe dans le moule afin de former les régions hydrophobes.

22. Procédé selon l'une quelconque des revendications 18 à 21, dans lequel ladite étape de dépôt (a) et/ou ladite étape de dépôt (b) est effectuée par un revêtement par centrifugation.

23. Procédé selon l'une quelconque des revendications 15 à 17, dans lequel le substrat possède une surface hydrophile formant une couche hydrophile, le procédé comprenant l'étape de :
dépôt des régions hydrophobes sur la couche hydrophile de manière à définir la pluralité de sites hydrophiles et la pluralité de pistes hydrophiles sur le substrat et à mettre en retrait les sites hydrophiles et les pistes hydrophiles entre les régions hydrophobes.

24. Procédé selon l'une quelconque des revendications 15 à 17, dans lequel le substrat possède une surface hydrophobe, le procédé comprenant :
la mise en retrait d'une pluralité de sites et d'une pluralité de pistes dans le substrat, en fournissant par ce moyen les sites et les pistes entre les régions hydrophobes ; et
le dépôt de matière hydrophile sur les sites et les pistes, en fournissant par ce moyen la pluralité de sites hydrophiles et la pluralité de pistes hydrophiles.

25. Procédé destiné à la formation d'un réseau cellulaire, le procédé comprenant :
la mise en contact d'une source de cellules à un dispositif selon l'une quelconque des revendications 1 à 13 ; et
le traitement des cellules afin de favoriser la formation d'un réseau cellulaire sur le dispositif.

26. Procédé selon la revendication 25, comprenant l'étape de traitement des cellules avec un milieu nutritif pour cellules afin de favoriser la formation du réseau cellulaire.

27. Procédé selon la revendication 25 ou 26, dans lequel la source de cellules est une source de neurones.

28. Procédé destiné à l'analyse d'un réseau cellulaire, le procédé comprenant l'utilisation d'une matrice d'électrodes pour étudier des caractéristiques des cellules d'un réseau cellulaire formé sur un dispositif selon l'une quelconque des revendications 1 à 13.

29. Procédé selon la revendication 28, dans lequel les cellules du réseau cellulaire sont des neurones.

30. Procédé de dosage destiné à identifier des substances qui affectent l'activité d'un réseau cellulaire, le procédé comprenant :
fournir à un réseau cellulaire une substance candidate, le réseau cellulaire étant formé sur un dispositif selon l'une quelconque des revendications 1 à 13 ; et
déterminer si la substance candidate affecte l'activité du réseau cellulaire.

31. Procédé de dosage selon la revendication 30, le procédé comprenant l'étape d'application de la substance candidate au réseau cellulaire après que le réseau cellulaire a été formé.

32. Procédé de dosage selon la revendication 30 ou 31, comprenant l'étape de comparaison de l'activité des cellules du réseau cellulaire à l'activité d'un réseau cellulaire témoin afin de déterminer si la substance candidate affecte l'activité du réseau cellulaire.

33. Procédé de dosage selon l'une quelconque des revendications 30 à 32, le procédé comprenant l'étape d'utilisation d'une matrice d'électrodes afin de déterminer si la substance candidate affecte l'activité du réseau cellulaire.

34. Procédé de dosage selon l'une quelconque des revendications 30 à 33, dans lequel le réseau cellulaire est un réseau de neurones.
